# FASCICULE DE BREVET EUROPEEN

(11) **EP 0 953 330 B1**
(45) Date de publication et mention de la délivrance du brevet: **11.05.2005**
(21) Numéro de dépôt: 99400663.3
(22) Date de dépôt: 18.03.1999
(51) Int. Cl.: A61K 7/02, A61K 7/00, A61K 7/043

(54) **Kit de maquillage associant un pigment goniochromatique et un pigment monocolore ayant une des couleurs du pigment goniochromatique, ses utilisations**
Schminkenkit mit einem goniochromatischen Pigment
Kit for make-up with a goniochromatical pigment

(30) Priorité: 10.04.1998 FR 9804581
(43) Date de publication de la demande: 03.11.1999
(73) Titulaire: L'OREAL, 75008 Paris (FR)
(72) Inventeur: Simon, Jean-Christophe, 75012 Paris (FR)
(74) Mandataire: Lhoste, Catherine

(56) Documents cités:
- EP-A- 0 815 826
- WO-A-91/13125
- DE-A- 4 240 743
- DE-A- 19 614 637
- FR-A- 2 750 601
- BASE DE DONN ES "CHEMICAL ABSTRACTS" (SERVEUR STN), Abrégé 124: 37 399, Colombus, OH, USA; & JP 07 267 820 A (SHISEIDO CO. Ltd) 17 OCTOBRE 1995 XP002090282

## Description

La présente invention se rapporte à un kit de maquillage destiné à un nouveau type de maquillage, associant un premier pigment goniochromatique et un second pigment notamment monocolore ayant une des couleurs du premier pigment. Ce kit comprend deux compositions cosmétiques de maquillage qui peuvent être appliquées sur la peau aussi bien du visage que du corps humain, sur les lèvres et sur les phanères comme les ongles, les cils, les sourcils ou les cheveux. L'invention a aussi pour objet un procédé de maquillage bicouche.

Chaque composition peut être une poudre libre ou compactée, un fond de teint, un fard à joues ou à paupières, un produit anti-cernes, un blush, un rouge à lèvres, un crayon à lèvres ou à yeux ou encore un vernis à ongles ou un produit de maquillage du corps.

Les compositions de maquillage sont constituées d'un véhicule approprié et de différents agents de coloration destinés à conférer une certaine couleur aux compositions avant et/ou après leur application sur la peau, les lèvres ou les phanères.

La gamme d'agents de coloration actuellement utilisée par les cosméticiens est assez limitée ; ces agents sont principalement des pigments organiques, des laques, des pigments minéraux ou des pigments nacrés. Les laques permettent d'obtenir des couleurs vives, mais sont pour la plupart instables à la lumière, à la température et au pH. Certaines présentent également l'inconvénient de tacher la peau de manière disgracieuse après application, par dégorgement du colorant. Les pigments minéraux, en particulier les oxydes minéraux sont au contraire très stables, mais donnent des couleurs plutôt ternes et pâles. Les pigments nacrés quant à eux permettent d'obtenir des couleurs variées, mais jamais intenses, à effets irisés mais le plus souvent assez faibles.

Pour pallier ces inconvénients, le demandeur a envisagé d'utiliser des pigments goniochromatiques, c'est-à-dire des pigments qui présentent des couleurs variant selon l'angle d'observation et l'incidence de la lumière, et confèrent des effets irisés un peu comme un produit nacré. On peut notamment se référer à la demande EP-A-815826, ou dans le cas de compositions capillaires, à FR 2 750 601.

Par ailleurs, certains produits de maquillage classiques permettent de créer des effets de décoration avec des motifs colorés : dessins, damiers, lettres etc. Cependant, ces motifs sont visibles selon tous les angles d'observation, ce qui rend le maquillage « statique ».

Les maquilleurs et les consommateurs recherchant de plus en plus des effets spéciaux et des couleurs originales, le demandeur a découvert un nouveau type de maquillage utilisant des pigments goniochromatiques. En utilisant un produit bicouche dont la sous-couche contient au moins un pigment goniochromatique, le demandeur a trouvé de façon surprenante qu'il était possible de tracer ou dessiner des motifs sur une telle couche (lettres, dessins, damiers...), en particulier avec un crayon ou pinceau, et que selon la direction d'observation, les motifs apparaissaient ou disparaissaient. L'invention permet un nouvel effet de mquillage : les motifs colorés apparaissent et disparaissent selon les mouvements de la personne maquillée. Le maquillage semble ainsi « vivant ».

De façon plus précise, l'invention a pour objet un kit de maquillage comprenant une première composition comprenant un milieu cosmétiquement acceptable et au moins un premier agent de coloration et une seconde composition comprenant un milieu cosmétiquement acceptable et au moins un second agent de coloration, l'un des agents étant un agent goniochromatique susceptible de produire différentes couleurs selon l'incidence de la lumière et l'angle d'observation, et l'autre un agent monocolore produisant une des couleurs de l'agent goniochromatique.

L'invention a encore pour objet un procédé de maquillage de la peau, des lèvres et/ou des phanères d'être humain, consistant à appliquer sur la peau, les lèvres et/ou les phanères une première couche d'une première composition comprenant un milieu cosmétiquement acceptable et au moins un premier agent de coloration puis à appliquer sur une partie de ladite première couche une seconde couche d'une seconde composition comprenant un milieu cosmétiquement acceptable et au moins un second agent de coloration, l'un des premier et second agents étant un agent goniochromatique susceptible de produire différentes couleurs selon l'incidence de la lumière et l'angle d'observation, l'autre agent étant un agent monocolore produisant une des couleurs de l'agent goniochromatique.

Dans le document WO-A-96/39307 de Flex Products, il est décrit une association de deux couches de motifs contenant chacune des pigments goniochromatiques, utilisable dans les encres et peintures pour voitures. Ce document ne prévoit pas d'utiliser cette association pour obtenir un maquillage original, ni de remplacer l'un des pigments goniochromatiques par des pigments classiques monocolores donnant une couleur identique à l'une des couleurs de la couche goniochromatique.

L'invention a aussi pour objet un produit cosmétique de maquillage comprenant un milieu cosmétiquement acceptable, au moins un premier agent de coloration et au moins un second agent de coloration, l'un des premier et second agents étant un agent goniochromatique susceptible de produire différentes couleurs selon l'incidence de la lumière et l'angle d'observation, l'autre étant un agent monocolore produisant une des couleurs de l'agent goniochromatique. De préférence, les premier et second agents de coloration sont conditionnés séparément dans respectivement des premières et secondes compositions cosmétiquement acceptables.

Avantageusement, ces deux compositions sont conditionnées dans des compartiments ou récipients distincts, accompagnés de moyens d'application appropriés.

Selon l'invention, la seconde couche est appliquée sur une partie seulement de la première couche. Elle peut être appliquée soit à l'une des extrémités de la première couche, soit au milieu, ou encore de façon discontinue notamment sous forme de motifs géométriques, symétriques ou dissymétriques (par exemple sous forme de points, carrés, ronds, étoiles, inscriptions alphanumériques ou tout autre symbole figuratif ou non), répartis de façon aléatoire ou ordonnée, à contours précis ou flous. Ainsi, selon un angle d'observation, notamment perpendiculaire à la seconde couche, les motifs de cette seconde couche disparaîtront car leur couleur sera identique à celle de la couche goniochromatique, et selon les autres directions, les motifs apparaîtront car de couleur différente de celle de la première couche.

Le premier agent de coloration est de préférence l'agent goniochromatique et le second agent est l'agent monocolore, toutefois l'inverse est possible.

Cette architecture bicouche peut être adaptée pour tous les produits de maquillage de la peau aussi bien du visage que du scalp et du corps, des muqueuses comme les lèvres et l'intérieur des paupières inférieures, et des phanères comme les ongles, les cils les cheveux, les sourcils, voire les poils. La seconde couche qui forme des motifs, peut être appliquée avec un stylo, crayon ou tout autre instrument (éponge, doigt, pinceau, brosse, plume etc.). Cette architecture peut aussi être appliquée sur les accessoires de maquillage comme les faux ongles, faux cils, perruques ou encore des pastilles ou des patchs adhérents sur la peau ou les lèvres (du type mouches).

L'invention se rapporte aussi à un support maquillé comprenant une première couche d'une première composition comportant au moins un premier agent de coloration et une seconde couche d'une seconde composition déposée en partie sur la première couche et comportant au moins un second agent de coloration, l'un des agents étant un agent goniochromatique susceptible de produire différentes couleurs selon l'incidence de la lumière et l'angle d'observation et l'autre agent étant un agent monocolore produisant une des couleurs de l'agent goniochromatique.

L'invention a aussi pour objet une composition cosmétique pour la mise en oeuvre du procédé de maquillage ci-dessus. Cette composition contient un milieu cosmétiquement acceptable et au moins un agent de coloration goniochromatique susceptible de produire différentes couleurs selon l'incidence de la lumière et l'angle d'observation, l'agent étant choisi parmi les structures multicouches interférentielles.

La première (ou la seconde) composition de l'invention peut comprendre un ou plusieurs agents de coloration goniochromatiques choisis parmi les agents de coloration mésomorphes ou à cristaux liquides (notés agents CL) et les structures multicouches interférentielles. De préférence, on utilise un seul agent goniochromatique pour une facilité de mise en oeuvre et un coût de fabrication réduit.

Les agents CL sont notamment des monomères ou polymères linéaires ou cycliques sur lesquels sont greffés des groupes mésomorphes, notamment cholestériques ou nématiques. Les agents de coloration CL comprennent par exemple des silicones ou des éthers de cellulose sur lesquels sont greffés des groupes mésomorphes. Les agents de coloration CL sont en particulier choisis parmi les organopolysiloxanes cycliques greffés par des groupements cholestériques et biphényliques. Ces organopolysiloxanes greffés sont en particulier réticulés selon une structure tridimensionnelle.

Les agents de coloration CL sont choisis notamment parmi les silicones cycliques de formule suivante : 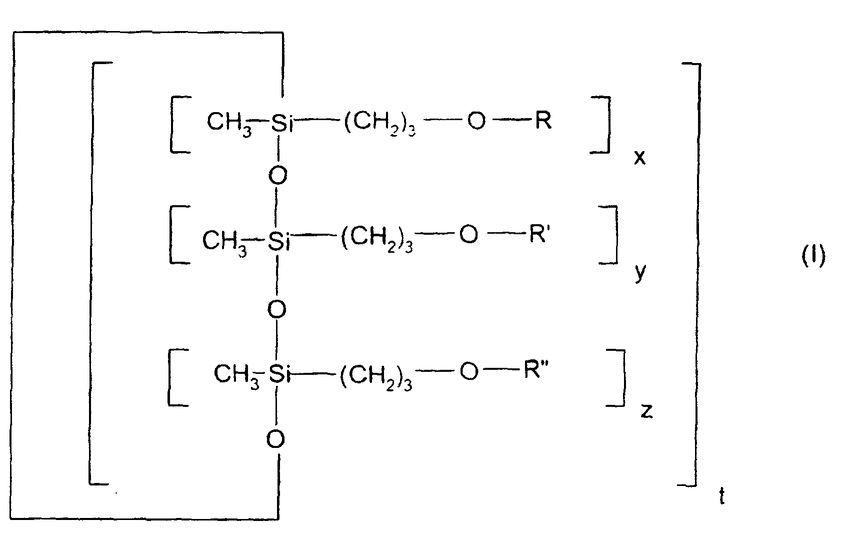 dans laquelle :
0 ≤ x ≤ 1 (de préférence 1) ; 0 ≤ y ≤ 1 (de préférence 1) ; 0 ≤ z ≤ 1 (de préférence 1) avec x + y + z ≠ 0 ; 3 ≤ t ≤ 10 ;
R désigne un groupe de formule suivante : 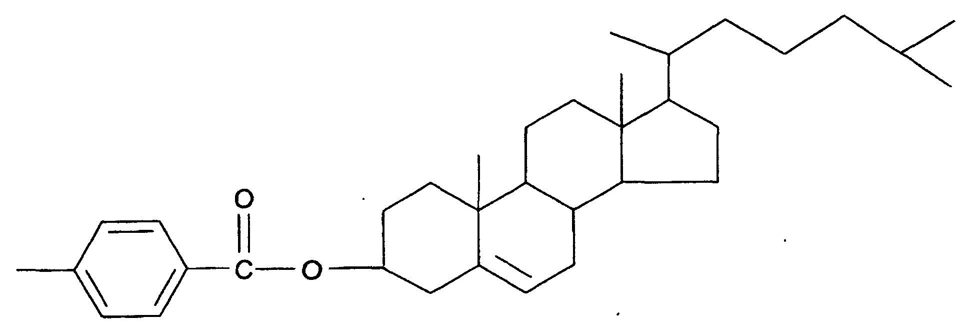
R' désigne un groupe de formule suivante : 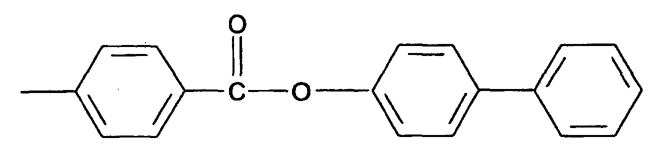
R" désigne un groupe de formule suivante :

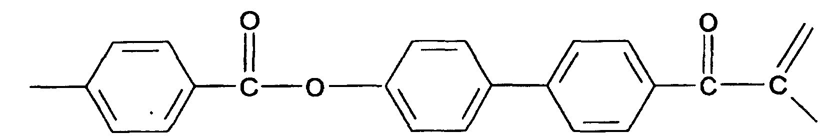

Ces composés se présentent généralement sous forme de poudres blanches amorphes. Ils ont la particularité de ne présenter l'effet de changement de couleur selon la direction d'observation qu'en fonction du support (et notamment de sa couleur) sur lequel on l'étale.

A titre d'exemples d'agent de coloration CL répondant à cette définition, on peut citer en particulier les « pigments CL » de la société Wacker dénommés SLM 41101 (bleu/vert), SLM 41102 (rouge/or) et SLM 41103 (jaune/vert), LC PIGMENT GRUN 516 S VP (bleu/vert).(Voir aussi le document EP-A-815826).

Les agents goniochromatiques à structures multicouches sont notamment ceux décrits dans les documents suivants : US-A-3 438 796, EP-A-227423, US-A-5 135 812, EP-A-170439, EP-A-341002, US-A-4 930 866, US-A- 5 641 719, EP-A-472371, EP-A-395410, EP-A-753545, EP-A-768343, EP-A-571836, EP-A-708154, EP-A-579091, US-A-5 411 586, US-A-5 364 467, WO-A-97/39066, DE-A-4 225 031, WO 9517479 (BASF), DE-A-196 14 637. Ils se présentent sous forme de paillettes, de couleur métallisée.

Les structures multicouches utilisables dans l'invention sont par exemple les structures suivantes : Al/SiO₂/Al/SiO₂/Al ; Cr/MgF₂/Al/MgF₂/Al ; MoS₂/SiO₂/Al/SiO₂/MoS₂ ; Fe₂O₃/SiO₂/Al/ SiO₂/Fe₂O₃ ; Fe₂O₃/SiO₂/Fe₂O₃/SiO₂/Fe₂O₃ ; MoS₂/SiO₂/mica-oxyde/SiO₂/MoS₂ ; Fe₂O₃/SiO₂/ mica-oxyde/SiO₂/Fe₂O₃. Selon l'épaisseur des différentes couches, on obtient différentes couleurs. Ainsi, avec la structure Fe₂O₃/SiO₂/Al/ SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.

On peut aussi utiliser des structures multicouches biréfringentes comprenant une alternance de couches polymériques du type naphtalate de polyéthylène et téréphtalate de polyéthylène, comme décrit dans le document WO-A-96/19347.

La seconde (ou première) composition de l'invention peut comprendre un ou plusieurs agents de coloration monocolores choisis parmi les colorants monocolores, les pigments monocolores et les nacres classiquement utilisés dans les compositions cosmétiques, et leurs associations. Selon l'invention, l'ensemble de ces agents doit présenter une des couleurs de l'agent de coloration goniochromatique ou de l'ensemble des agents goniochromatiques.

Par pigments, il faut comprendre des particules blanches ou colorées, minérales ou organiques, insolubles dans la phase grasse liquide, destinées à colorer et/ou opacifier la seconde composition. Par nacres, il faut comprendre des particules irisées, notamment produites par certains mollusques dans leur coquille ou bien synthétisées. Par colorants, il faut comprendre des composés généralement organiques solubles dans les corps gras comme les huiles ou dans une phase hydroalcoolique.

Les agents de coloration des première et seconde couches peuvent être présents à raison de 0,01 à 60 % du poids total respectivement de la première composition et seconde composition, de préférence de 0,05 à 30 % et plus particulièrement de 1 à 20 %, pour des compositions non pulvérulentes. Pour des compositions pulvérulentes, la quantité d'agents de coloration peut aller jusqu'à 85 % et même jusqu'à 98 %.

Comme pigments minéraux monocolores utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium, aluminium.

Les colorants peuvent être liposolubles ou hydrosolubles. Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter de 0,01 à 20 % du poids total de la seconde composition et mieux de 0,1 à 10 %. Les colorants hydrosolubles sont notamment le sulfate de cuivre, de fer, des sulfopolyesters hydrosolubles tels que ceux décrits dans les documents FR-96 154152, les rhodamines, les colorants naturels (carotène, jus de betterave), bleu de méthylène.

Les nacres peuvent être présentes dans la seconde composition à raison de 0 à 20 % du poids total de ladite seconde composition, de préférence à un taux de l'ordre de 1 à 15 %. Parmi les nacres utilisables dans la seconde composition, on peut citer le mica recouvert d'oxyde de titane, d'oxyde de fer, de pigment naturel ou d'oxychlorure de bismuth tel que le mica titane coloré.

Les première et seconde compositions selon l'invention peuvent, de plus, comprendre tous les ingrédients classiquement utilisés dans les domaines concernés et plus spécialement dans les domaines cosmétique et dermatologique. Ces ingrédients sont en particulier choisis parmi les conservateurs, les stabilisants, les neutralisants, les épaississants de phase aqueuse (biopolymères polysaccharidiques, les polymères synthétiques) ou grasse comme les argiles, les charges, les parfums, les actifs hydrophiles ou lipophiles, les tensioactifs, les antioxydants, les polymères filmogènes et leurs mélanges. Les quantités de ces différents ingrédients sont celles classiquement utilisées dans les domaines concernés et par exemple de 0,01 % à 30 % du poids total de la composition. La nature de ces ingrédients et leur proportion doivent être compatibles avec l'obtention de compositions selon l'invention, stables épaissies et brillantes. La composition peut aussi contenir de l'eau à une concentration allant de 0 à 95% du poids total de la composition ou des solvants organiques pouvant représenter jusqu'à 90%.

Par charges, il faut comprendre des particules incolores ou blanches, minérales ou de synthèse, lamellaires ou non lamellaires. Ces charges peuvent être introduites dans les première ou seconde couches en vue de notamment modifier la texture de ces compositions. Elles peuvent être présentes à raison de 0 à 35 % du poids total de la chaque composition, de préférence 0,5 à 15 %. On peut notamment citer le talc, le stéarate de zinc, le mica, le kaolin, les poudres de Nylon (Orgasol notamment) et de polyéthylène, le Téflon, l'amidon, le nitrure de bore, des microsphères de copolymères telles que l'Expancel (Nobel Industrie), le polytrap (Dow Corning) et les microbilles de résine de silicone (Tospearl de Toshiba, par exemple) ou encore la silice.

Les première et seconde compositions de l'invention comprennent, avantageusement une phase grasse contenant des corps gras liquides, solides ou pâteux à température ambiante. Les corps gras solides à température ambiante permettent de structurer la composition ; ils sont choisis parmi les gommes et/ ou les cires. Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.

Comme cire utilisable dans les première et seconde compositions de l'invention, on peut citer, la lanoline, la cire d'abeilles, la cire de Carnauba ou de Candellila, la paraffine, les cires de lignite ou microcristalline, la cérésine ou l'ozokérite ; les cires synthétiques comme les cires de polyéthylène, les cires de Fischer Tropsch, les cires de silicones comme les alkyl ou alkoxy-diméticone ayant de 16 à 45 atomes de carbone.

Les gommes sont notamment des organopolysiloxanes ayant un poids moléculaire moyen de 1000 à 500 000.

La nature et la quantité de ces gommes ou cires sont fonction des propriétés mécaniques et de textures recherchées. A titre indicatif, chaque composition peut contenir de 0 à 50 % en poids de cires, par rapport au poids total de la composition et mieux de 5 à 30 %.

Comme corps gras liquide à température, utilisable dans les compositions de l'invention, on peut citer :
- les huiles hydrocarbonées d'origine animale telles que le perhydrosqualène ;
- les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stéarineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les isoparaffines comme l'isohexadécane et l'isodécane ;
- les esters et les éthers de synthèse notamment d'acides gras comme les huiles de formule R₁COOR₂ dans laquelle R₁ représente le reste d'un acide gras supérieur comportant de 7 à 29 atomes de carbone et R₂ représente une chaîne hydrocarbonée contenant de 3 à 30 atomes de carbone comme par exemple l'huile de Purcellin, le myristate d'isopropyle, le palmitate d'éthyl-2hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ;
- des alcools gras ayant de 12 à 26 atomes de carbone comme l'octyldodécanol, le 2-butyloctanol, le 2-hexyldécanol, le 2-undécylpentadécanol, l'alcool oléique ;
- les huiles fluorées partiellement hydrocarbonées et/ou siliconées comme celle décrites dans le document JP-A-2-295912 ;
- les huiles siliconées comme les polyméthylsiloxanes (PDMS) volatiles ou non, linéaires ou cycliques, liquides ou pâteux à température ambiante ; les silicones phénylées comme les phényl triméthicones, les diphényl diméthicones, les phényl diméthicones, les phényltriméthylsiloxy diphényl siloxanes ;
- les huiles fluorées et les huiles fluorosiliconées ;
- leurs mélanges.

Ces huiles peuvent représenter de 0 à 99,9% du poids total de chaque composition.

Comme huile de silicone volatile utilisable dans l'invention, on peut citer les silicones linéaires ou cycliques ayant de 2 à 7 atomes de silicium, ces silicones comportant éventuellement des groupes alkyle ayant de 1 à 10 atomes de carbone. Ainsi, ces silicones sont notamment l'hexaméthyldisiloxane, la cyclopenta- ou cyclotétra- ou cyclohexa-diméthylsiloxane. Ces huiles volatiles peuvent représenter de 0 à 50% du poids total de la composition.

Comme solvant utilisable dans l'invention, on peut citer :
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde.

Ces solvants conviennent plus particulièrement pour le maquillage et le soin des ongles : la composition constitue alors un vernis à ongles ou un produit de soin des ongles. Comme solvant on peut aussi utiliser l'eau et les milieux hydroalcooliques.

Comme polymère filmifiable utilisable dans l'invention, on peut citer la nitrocellulose, l'acétobutyrate de cellulose, les butyralpolyvinyliques, les résines alkydes, les résines résultant de la condensation de formaldéhyde avec une arylsulfonamide, les polyesters, les polyuréthannes, les polyester-polyuréthanes, les polyéther-polyuréthanes, les polymères radicalaires, notamment de type acrylique, acrylique styrène et/ou vinylique et leurs mélanges.

Les polymères peuvent être dissous ou dispersés dans la composition. Ils peuvent être généralement présents à une teneur allant de 0,5 % à 40 % en poids par rapport au poids total de la composition, et mieux allant de 10 % à 20 % en poids.

La composition selon l'invention peut également comprendre, en plus du ou des polymères filmogènes, des agents plastifiants qui permettent de régler la flexibilité du film de polymère sans affaiblir sa résistance physique.
Les agents plastifiants utilisables sont ceux couramment employés dans les compositions de vernis à ongles. Comme plastifiants, on peut citer les phtalates de dibutyle, de dioctyle, de di-isobutyle, de diméthoxyéthyl, les benzoates de benzyle, de glycéryle ; les citrates de triéthyle, de tributyle, l'acétyl-citrate de tributyle ; les phosphates de tributyle, de triphényle ; les glycols ; le camphre ainsi que leurs dérivés et leurs mélanges. Les plastifiants peuvent être généralement présents à une teneur allant de 1 % à 30 % en poids par rapport au poids total de la composition, et mieux de 5 % à 10 % en poids.

Les compositions de l'invention peuvent se présenter sous toutes les formes galéniques normalement utilisées pour une application topique, et notamment sous forme d'une solution huileuse ou aqueuse, d'un gel huileux ou aqueux, d'une émulsion huile-dans-eau ou eau-dans-huile, d'une dispersion d'huile dans de l'eau grâce à des vésicules, les vésicules étant situées à l'interface huile/eau ou encore d'une poudre. Chaque composition peut avoir l'aspect d'une lotion, d'une crème, d'une pommade, d'une pâte souple, d'un onguent, d'un solide coulé ou moulé et notamment de stick ou de coupelle, ou encore de solide compacté.

Le produit selon l'invention peut être avantageusement utilisé pour le maquillage de la peau et/ou des muqueuses et/ou des phanères selon la nature des ingrédients utilisés. En particulier, chaque composition de l'invention peut être sous forme de bâton ou pâte de rouge à lèvres, de fond de teint solide, de produit anti-cernes ou contours des yeux, d'eye liner, de mascara, d'ombre à paupières, de vernis à ongles à milieu solvant ou aqueux, de produit de maquillage du corps ou encore un produit de coloration de la peau. Ces compositions peuvent, en outre, contenir des actifs cosmétiques ou dermatologiques, en vue, notamment d'apporter un aspect soin ou traitant à la composition. Ainsi ces compositions peuvent contenir des vitamines et autres actifs lipophiles (lanoline, filtre UVA) ou hydrophiles (hydratants comme la glycérine).

L'invention a encore pour objet un produit à èvres, un fond de teint, un tatouage, un vernis à ongles, un fard à joues ou à paupières cortenant un milieu cosmétiquement acceptable et des première et seconde compositions telles que décrites précédemment.

L'invention a aussi pour objet l'utilisation du produit cosmétique ci-dessus pour faire apparaître ou disparaître sur la peau et/ou les lèvres et/ou les phanères d'êtres humains des motifs selon l'angle d'observation.

Les compositions de l'invention peuvent être obtenues par chauffage des différents constituants à la température de fusion des cires les plus élevées, puis coulage du mélange fondu dans un moule (coupelle ou doigt de gant). Elles peuvent aussi être obtenues par extrusion comme décrit dans la demande EP-A-667 146.

Les exemples de composition ci-après sont donnés à titre illustratif. Les quantités y sont données en % en poids.

### Exemple de réalisation du procédé et du produit selon l'invention: Vernis à ongle

a) La base de vernis (appelée base VAO) utilisée pour les deux couches est la suivante :
   - Polyuréthane aliphatique (filmogène) 33,9 g
   - N-méthyl pyrrolidone (stabilisant) 8,2 g
   - Tri-éthylamine (neutralisant) 1,9 g
   - Acétyl-citrate de tri-butyle 3,2 g
   - Eau qsp 100 g
b) La composition pour former la première couche 1 de vernis à ongles est réalisée avec :
   - Base VAO 95 g
   - Pigments LC Wacker LC PIGMENT GRUN 516 S VP 5 g

   Cette composition est obtenue en mélangeant à température ambiante la base VAO et les pigments sous agitation. Sa couleur change selon l'angle d'observation et varie du bleu foncé au vert foncé. Cette composition est appliquée en continue sur des ongles démaquillés, sous forme de monocouche.
c) La composition pour former la seconde couche 2 de vernis à ongles est composée de :
   - Base VAO 90,00 g
   - Dispersion aqueuse a 60% de dioxyde de titane (ci: 77891) dans mélange eau/glycérol/tensioactif (16/18/5) 4,73 g
   - Dispersion de carmin permanent FB à 40% dans mélange eau/glycérol/lauryl éther sulfate de sodium (29,7/25/5) 0,13 g
   - Dispersion aqueuse à 42% de jaune Hansa G (ci: 11680) dans mélange eau/glycérol/lauryl éther sulfate de sodium (27,7/25/5) 1.51 g
   - Dispersion de vert de phtalocyanine FB à 48% (ci: 74260) dans mélange eau/glycérol/lauryl éther sulfate de sodium) (21,7/25/5) 3.63 g
   - Conservateurs qs

La composition de la couche 2 possède une couleur verte qui est identique à l'une des la couleur de la composition de la couche 1. Cette composition est obtenue en mélangeant les pigments et la base VAO comme on le fait classiquement pour la fabrication d'un vernis.

Cette composition est appliquée au pinceau sur la couche 1 en formant des motifs ( tâches, étoiles, papillons). Après séchage du vernis bicouche, l'observation dans une direction perpendiculaire à sa surface (ou surface de l'ongle) ne fait apparaître qu'une couleur homogène verte de l'ongle et une observation dans une autre direction fait apparaître des motifs de couleur verte sur fond bleu.

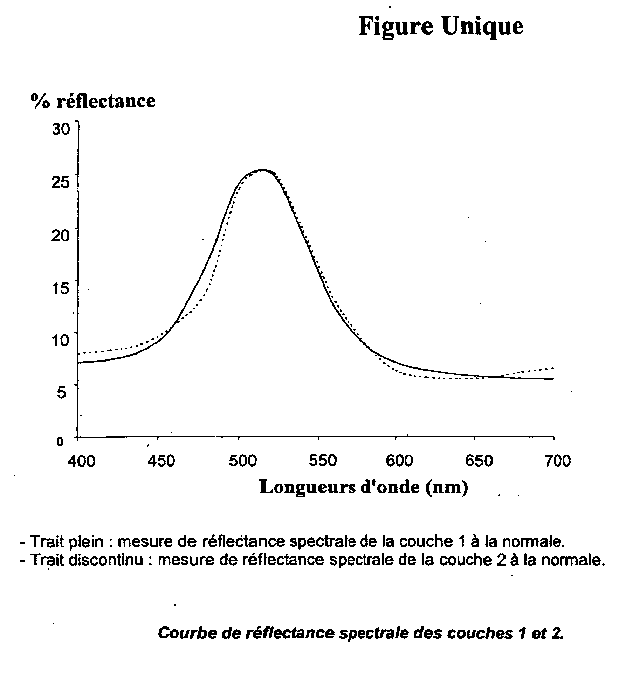

Sur la figure annexée on a représenté la réflectance des couches de vernis 1 et 2, exprimée en % (% de lumière transmise) en fonction de la longueur d'onde, exprimée en nm. Cette réflectance a été mesurée selon une observation perpendiculaire aux couches 1 et 2. La courbe en trait plein est la réflectance de la couche 1 et la courbe en tirets est la réflectance de la couche 2.

Ces courbes indiquent clairement que la couleur obtenue pour les deux couches est la même, dans la direction perpendiculaire aux couches, et qu'il n'y a pas de métamérisme, c'est-à-dire de changement de couleur selon la nature de l'éclairement. Ainsi, le maquillage vu en lumière naturelle sera identique à celui vu en lumière artificielle.

## Revendications

1. Produit cosmétique de maquillage comprenant un milieu cosmétiquement acceptable, au moins un premier agent de coloration et au moins un second agent de coloration, l'un des agents étant un agent goniochromatique susceptible de produire différentes couleurs selon l'incidence de la lumière et l'angle d'observation choisi parmi les structures multicouches interférentielles et l'autre agent étant un agent monocolore produisant une des couleurs de l'agent goniochromatique.

2. Produit selon l'une des revendications 1, **caractérisé en ce que** l'agent goniochromatique comporte une structure multicouche interférentielle choisie parmi les structures Al/SiO₂/Al/SiO₂/Al ; Cr/MgF₂/Al/MgF₂/Al ; MoS₂/SiO₂/Al/SiO₂/MoS₂ ; Fe₂O₃/SiO₂/Al/SiO₂/Fe₂O₃ ; Fe₂O₃/SiO₂/Fe₂O₃/SiO₂/Fe₂O₃ ; MoS₂/SiO₂/mica-oxyde/SiO₂/MoS₂ ; Fe₂O₃/SiO₂/mica-oxyde/SiO₂/Fe₂O₃.

3. Produit selon l'une des revendications précédentes, **caractérisé en ce que** l'agent de coloration monocolore est choisi parmi les colorants monocolores, les pigments monocolores et/ou les nacres.

4. Produit selon l'une des revendications précédentes, **caractérisé en ce que** l'agent de coloration monocolore est choisi parmi les oxydes de titane, de zirconium ou de cérium ainsi que les oxydes de zinc, de fer ou de chrome, le bleu ferrique, le noir de carbone, et les laques de baryum, de strontium, de calcium, ou d'aluminium, le rouge Soudan, le DC Red 17, le DC Green 6, le β-carotène, l'huile de soja, le brun Soudan, le DC Yellow, 11, le DC Violet 2, le DC orange 5, le jaune quinoléine, le mica recouvert d'oxyde de titane, d'oxyde de fer, de pigment naturel ou d'oxychlorure de bismuth tel que le mica titane coloré.

5. Produit selon l'une des revendications précédentes, **caractérisé en ce que** chaque agent de coloration représente de 0,01 à 98 % et mieux de 0,05 à 85 % du poids total du produit.

6. Produit selon l'une des revendications précédentes, **caractérisé en ce qu'**il est conditionné sous forme de fond de teint, vernis à ongle, produit de maquillage du corps ou des lèvres, fard à joues ou à paupières.

7. Produit selon l'une des revendications précédentes, **caractérisé en ce** les premier et second agents de coloration sont conditionnés dans respectivement des première et seconde compositions contenant chacune un milieu cosmétiquement acceptable.

8. Produit selon l'une des revendications précédentes, **caractérisé en ce qu'**il est sous forme d'une solution huileuse ou aqueuse, d'un gel huileux ou aqueux, d'une émulsion huile-dans-eau ou eau-dans-huile, d'une dispersion d'huile dans de l'eau grâce à des vésicules, les vésicules étant situées à l'interface huile/eau, ou d'une poudre.

9. Produit selon l'une des revendications précédentes, **caractérisé en ce que** le milieu cosmétiquement acceptable contient, en outre, au moins un ingrédient choisi parmi les huiles, les solvants, les cires, les polymères filmogènes, les charges, les actifs hydrophiles ou lipophiles, les épaississants de phase aqueuse ou grasse, les tensioactifs, les antioxydants, les parfums, les plastifiants, les neutralisants, les stabilisants.

10. Procédé de maquillage de la peau, des lèvres et/ou des phanères d'être humain, consistant à appliquer sur la peau, les lèvres et/ou les phanères une première couche d'une première composition comprenant un milieu cosmétiquement acceptable et au moins un premier agent de coloration puis à appliquer sur une partie seulement de ladite première couche une seconde couche d'une seconde composition comprenant un milieu cosmétiquement acceptable et au moins un second agent de coloration, l'un des agents étant un agent goniochromatique susceptible de produire différentes couleurs selon l'incidence de la lumière et l'angle d'observation et l'autre agent étant un agent monocolore produisant une des couleurs de l'agent goniochromatique.

11. Procédé selon la revendication 10, **caractérisé en ce que** l'agent de coloration goniochromatique est choisi parmi les agents de coloration mésomorphes ou à cristaux liquides et les structures multicouches interférentielles.

12. Procédé selon la revendication 10 ou 11, **caractérisé en ce que** l'agent de coloration goniochromatique est choisi parmi les polymères linéaires ou cycliques sur lesquels sont greffés des groupes mésomorphes.

13. Procédé selon l'une des revendications 10 à 12, **caractérisé en ce que** l'agent de coloration goniochromatique est choisi parmi les polymères siliconés et les éthers de cellulose sur lesquels sont greffés des groupes cholestériques et biphényliques.

14. Procédé selon l'une des revendications 10 à 13, **caractérisé en ce que** l'agent de coloration goniochromatique comporte une structure siliconée de formule suivante : 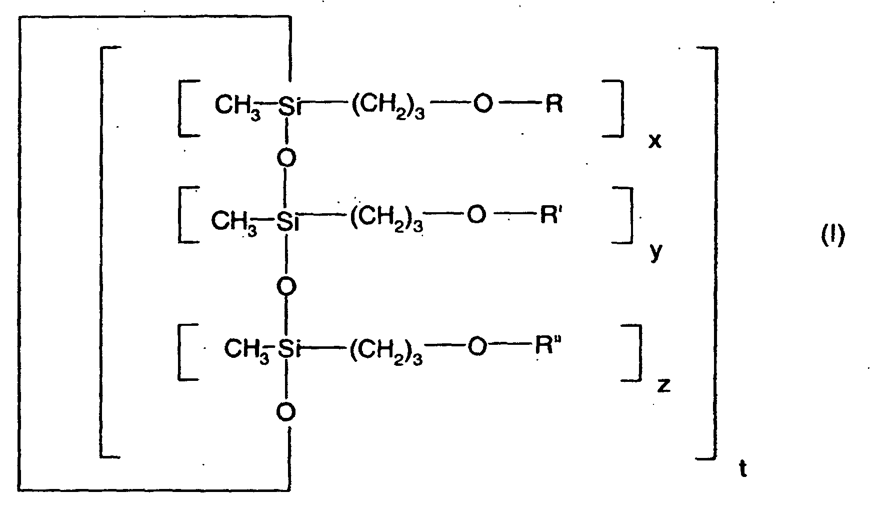 dans laquelle :
0 ≤ x ≤ 1 (de préférence 1) ; 0 ≤ y ≤ 1 (de préférence 1) ; 0 ≤ z ≤ 1 (de préférence 1) avec x + y +z ≠ 0; 3 ≤ t ≤ 10 ;
R désigne un groupe de formule suivante : 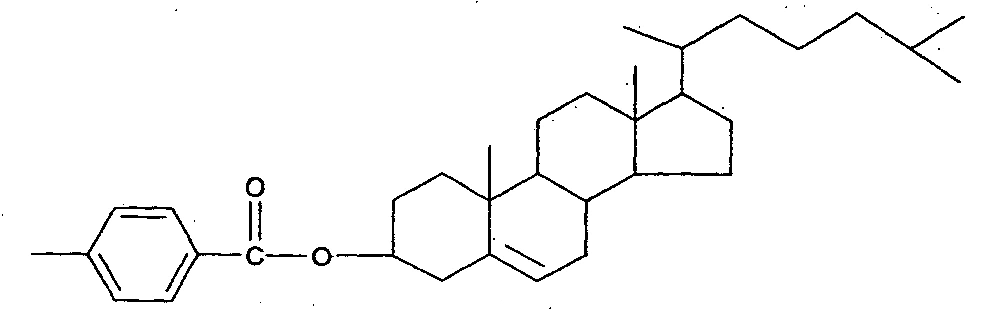
R' désigne un groupe de formule suivante : 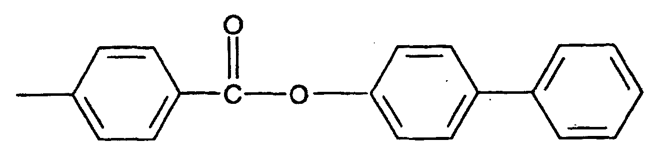
R" désigne un groupe de formule suivante :

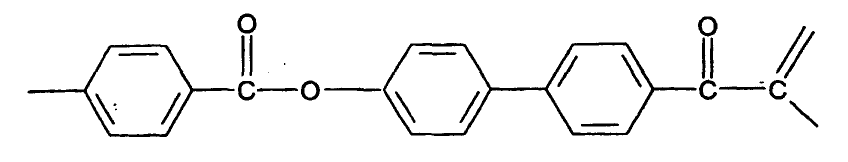

15. Procédé selon l'une des revendications 10 et 11, **caractérisé en ce que** l'agent goniochromatique comporte une structure multicouche interférentielle, choisie parmi les structures Al/SiO₂/Al/SiO₂/Al ; Cr/MgF₂/Al/MgF₂/Al ; MoS₂/SiO₂/Al/SiO₂/MoS₂ ; Fe₂O₃/SiO₂/Al/SiO₂/Fe₂O₃ ; Fe₂O₃/SiO₂/Fe₂O₃/SiO₂/Fe₂O₃ ; MoS₂/SiO₂/mica-oxyde/SiO₂/MoS₂ ; Fe₂O₃/SiO₂/mica-oxyde/SiO₂/Fe₂O₃.

16. Procédé selon l'une des revendications 10 à 15, **caractérisé en ce que** l'agent de coloration monocolore est choisi parmi les colorants monocolores, les pigments monocolores et/ou les nacres.

17. Procédé selon l'une des revendications 10 à 16, **caractérisé en ce que** l'agent de coloration monocolore est choisi parmi les oxydes de titane, de zirconium ou de cérium ainsi que les oxydes de zinc, de fer ou de chrome, le bleu ferrique, le noir de carbone, et les laques de baryum, de strontium, de calcium, ou d'aluminium, le rouge Soudan, le DC Red, le DC Green 6, le β-carotène, l'huile de soja, le brun Soudan, le DC Yellow, 11, le DC Violet 2, le DC orange 5, le jaune quinoléine, le mica recouvert d'oxyde de titane, d'oxyde de fer, de pigment naturel ou d'oxychlorure de bismuth tel que le mica titane coloré.

18. Procédé selon l'une des revendications 10 à 17, **caractérisé en ce que** chaque agent de coloration représente de 0,01 à 98 % et mieux de 0,05 à 85 % du poids total du produit.

19. Procédé selon l'une des revendications 10 à 18, **caractérisé en ce que** chaque première et seconde composition est conditionnée sous forme de fond de teint, vernis à ongle, produit de maquillage du corps ou des lèvres, fard à joues ou à paupières.

20. Procédé selon l'une des revendications 10 à 19, **caractérisé en ce que** les première et seconde compositions sont sous forme d'une solution huileuse ou aqueuse, d'un gel huileux ou aqueux, d'une émulsion huile-dans-eau ou eau-dans-huile, d'une dispersion d'huile dans de l'eau grâce à des vésicules, les vésicules étant situées à l'interface huile/eau, ou d'une poudre.

21. Procédé selon l'une des revendications 10 à 20, **caractérisé en ce que** le milieu cosmétiquement acceptable contient, en outre, au moins un ingrédient choisi parmi les huiles, les solvants, les cires, les polymères filmogènes, les charges, les actifs hydrophiles ou lipophiles, les épaississants de phase aqueuse ou grasse, les tensioactifs, les antioxydants, les parfums, les plastifiants, les neutralisants, les stabilisants.

22. Procédé selon l'une des revendications 10 à 21, **caractérisé en ce que** la seconde couche est discontinue.

23. Procédé selon l'une des revendications 10 à 22, **caractérisé en ce que** la seconde couche comprend des motifs.

24. Procédé selon l'une des revendications 10 à 23, **caractérisé en ce que** la seconde coche contient des motifs répartis de façon aléatoire ou ordonnée, de forme symétrique ou dissymétrique.

25. Kit de maquillage comprenant une première composition comprenant un milieu cosmétiquement acceptable et au moins un premier agent de coloration et une seconde composition comprenant un milieu cosmétiquement acceptable et au moins un second agent de coloration, l'un étant un agent goniochromatique susceptible de produire différentes couleurs selon l'incidence de la lumière et l'angle d'observation choisi parmi les structures multicouches interférentielles et l'autre un agent monocolore produisant une des couleurs de l'agent goniochromatique.

26. Kit selon la revendication 25, **caractérisé en ce que** les premier et second agent de coloration sont conditionnés séparément dans respectivement des premières et secondes compositions contenant un milieu cosmétiquement acceptable.

27. Kit selon la revendication 25 ou 26, **caractérisé en ce que** l'agent de coloration à structure multicouche est choisi parmi les structures Al/SiO₂/Al/SiO₂/Al ; Cr/MgF₂/Al/MgF₂/Al ; MoS₂/SiO₂/Al/SiO₂/MoS₂ ; Fe₂O₃/SiO₂/Al/ SiO₂/Fe₂O₃ ; Fe₂O₃/SiO₂/Fe₂O₃/SiO₂/Fe₂O₃ ; MoS₂/SiO₂/mica-oxyde/SiO₂/MoS₂ ; Fe₂O₃/SiO₂/ mica-oxyde/SiO₂/Fe₂O₃.

28. Kit selon l'une des revendications 25 à 27, **caractérisé en ce que** l'agent de coloration monocolore est choisi parmi les colorants, les pigments et/ou les nacres.

29. Kit selon l'une des revendications 25 à 28, **caractérisé en ce que** l'agent de coloration monocolore est choisi parmi les oxydes de titane, de zirconium ou de cérium ainsi que les oxydes de zinc, de fer ou de chrome, le bleu ferrique, le noir de carbone, et les laques de baryum, de strontium, de calcium, ou d'aluminium, le rouge Soudan, le DC Red 17, le DC Green 6, le β-carotène, l'huile de soja, le brun Soudan, le DC Yellow, 11, le DC Violet 2, le DC orange 5, le jaune quinoléine, le mica recouvert d'oxyde de titane, d'oxyde de fer, de pigment naturel ou d'oxychlorure de bismuth tel que le mica titan coloré.

30. Kit selon l'une des revendications 25 à 29, **caractérisé en ce qu'**il contient des moyens pour appliquer sur la peau et/ou les muqueuses et/ou les phanères la première et la seconde compositions.

31. Kit selon la revendication 30, **caractérisé en ce que** les moyens sont choisis parmi les pinceaux, stylos, brosses, plumes, crayons.

32. Kit selon l'une des revendications 25 à 31, **caractérisé en ce que** les première et seconde compositions sont conditionnées dans des compartiments ou récipients distincts.

33. Kit selon l'une des revendications 25 à 32, **caractérisé en ce que** le milieu cosmétiquement acceptable contient, en outre, au moins un ingrédient choisi parmi les huiles, solvants, cires, polymères filmogènes, charges, actifs hydrophiles ou lipophiles, épaississants de phase aqueuse ou grasse, tensioactifs, antioxydants, parfums, plastifiants, neutralisants, stabilisants.

34. Kit selon l'une des revendications 25 à 33, **caractérisé en ce que** les compositions sont sous forme de solution huileuse ou aqueuse, de gel huileux ou aqueux, d'émulsion huile-dans-eau ou eau-dans-huile, de poudre ou de dispersion d'huile dans eau grâce à des vésicules, ces vésicules étant situées à l'interface huile/eau.

35. Kit selon l'une des revendications 25 à 34, **caractérisé en ce que** les compositions sont sous forme de fond de teint, vernis à ongle, produit de maquillage du corps ou des lèvres, fard à joues ou à paupières.

36. Utilisation du produit selon l'une des revendications 1 à 9 pour faire apparaître ou disparaître sur la peau et/ou les lèvres et/ou les phanères d'êtres humains des motifs de couleur selon l'angle d'observation.

37. Support maquillé comprenant une première couche d'une première composition comportant au moins un premier agent de coloration et une seconde couche d'une seconde composition déposée en partie seulement sur la première couche et comportant au moins un second agent de coloration, l'un des agents étant un agent goniochromatique susceptible de produire différentes couleurs selon l'incidence de la lumière et l'angle d'observation et l'autre agent étant un agent monocolore produisant une des couleurs de l'agent goniochromatique.

38. Support selon la revendication 37, **caractérisé en ce qu'**il se présente sous forme de faux ongles, faux cils, perruques.

39. Support selon la revendication 37 ou 38, **caractérisé en ce** la seconde couche est discontinue.

40. Support selon l'une des revendications 37 à 39, **caractérisé en ce que** la seconde couche comprend des motifs répartis de façon aléatoire ou ordonnée.

## Patentansprüche

1. Kosmetisches Produkt zum Schminken, das ein kosmetisch akzeptables Medium, mindestens ein erstes Farbmittel und mindestens ein zweites Farbmittel enthält, wobei ein Farbmittel ein goniochromatischer Stoff ist, der in Abhängigkeit vom Einfallswinkel des Lichts und vom Betrachtungswinkel verschiedene Farben erzeugen kann und unter den mehrschichtigen Interferenzstrukturen ausgewählt ist, und das andere Farbmittel ein einfarbiger Stoff ist, der eine der Farben des goniochromatischen Stoffes erzeugt.

2. Produkt nach Anspruch 1, **dadurch gekennzeichnet, dass** der goniochromatische Stoff, der eine mehrschichtige Interferenzstruktur aufweist, unter den folgenden Strukturen ausgewählt ist: Al/SiO₂/Al/SiO₂/Al; Cr/MgF₂/Al/MgF₂/Al; MoS₂/SiO₂/Al/SiO₂/MoS₂; Fe₂O₃/SiO₂/Al/SiO₂/Fe₂O₃; Fe₂O₃/SiO₂/Fe₂O₃/SiO₂/Fe₂O₃; MoS₂/SiO₂/Glimmer-Oxid/SiO₂/MoS₂; Fe₂O₃/SiO₂/Glimmer-Oxid/SiO₂/Fe₂O₃.

3. Produkt nach einem der vorhergehenden Ansprüche, **dadurch gekennzeichnet, dass** das einfarbige Farbmittel unter den einfarbigen Farbstoffen, einfarbigen Pigmenten und/oder Perlglanzpigmenten ausgewählt ist.

4. Produkt nach einem der vorhergehenden Ansprüche, **dadurch gekennzeichnet, dass** der einfarbige Farbstoff unter den Oxiden von Titan, Zirconium oder Cer sowie den Oxiden von Zink, Eisen oder Chrom, Eisenblau, Ruß und den Lacken von Barium, Strontium, Calcium oder Aluminium, Sudanrot, DC Red 17, DC Green 6, β-Carotin, Sojaöl, Sudanbraun, DC Yellow 11, DC Violet 2, CD Orange 5, Chinolingelb, und mit Titanoxid, Eisenoxid, natürlichem Pigment oder Bismutoxidchlorid überzogenem Glimmer, beispielsweise farbigen Titanglimmerpigmenten, ausgewählt ist.

5. Produkt nach einem der vorhergehenden Ansprüche, **dadurch gekennzeichnet, dass** jedes Farbmittel 0,01 bis 98 % und besser 0,05 bis 85 % des Gesamtgewichts des Produkts ausmacht.

6. Produkt nach einem der vorhergehenden Ansprüche, **dadurch gekennzeichnet, dass** es als Make-up, Nagellack, Produkt zum Schminken des Körpers oder der Lippen, Wangenrouge oder Lidschatten konfektioniert ist.

7. Produkt nach einem der vorhergehenden Ansprüche, **dadurch gekennzeichnet, dass** das erste Farbmittel und das zweite Farbmittel in einer ersten bzw. einer zweiten Zusammensetzung enthalten sind, die jeweils ein kosmetisch akzeptables Medium enthalten.

8. Produkt nach einem der vorhergehenden Ansprüche, **dadurch gekennzeichnet, dass** es als ölige oder wässrige Lösung, öliges oder wässriges Gel, Öl-in-Wasser-Emulsion oder Wasser-in-Öl-Emulsion, Dispersion eines Öls in Wasser mit Hilfe von Vesikeln, wobei sich die Vesikel an der Grenzfläche Öl/Wasser befinden, oder als Pulver vorliegt.

9. Produkt nach einem der vorhergehenden Ansprüche, **dadurch gekennzeichnet, dass** das kosmetisch akzeptable Medium ferner mindestens einen Bestandteil enthält, der unter den Ölen, Lösungsmitteln, Wachsen, filmbildenden Polymeren, Füllstoffen, hydrophilen oder lipophilen Wirkstoffen, Verdickungsmitteln für die wässrige Phase oder die Fettphase, grenzflächenaktiven Stoffen, Antioxidantien, Parfums, Weichmachern, Neutralisationsmitteln und Stabilisatoren ausgewählt ist.

10. Verfahren zum Schminken der menschlichen Haut, der Lippen und/oder der Hautanhangsgebilde, das darin besteht, auf die Haut, die Lippen und/oder die Hautanhangsgebilde eine erste Schicht einer ersten Zusammensetzung, die ein kosmetisch akzeptables Medium und mindestens ein erstes Farbmittel enthält, und anschließend auf nur einen Teil der ersten Schicht eine zweite Schicht einer zweiten Zusammensetzung aufzutragen, die ein kosmetisch akzeptables Medium und ein zweites Farbmittel enthält, wobei ein Farbmittel ein goniochromatischer Stoff ist, der in Abhängigkeit vom Einfallswinkel des Lichts und dem Betrachtungswinkel unterschiedliche Farben erzeugen kann, und das andere Farbmittel ein einfarbiger Stoff ist, der eine der Farben des goniochromatischen Stoffes erzeugt.

11. Verfahren nach Anspruch 10, **dadurch gekennzeichnet, dass** das goniochromatische Farbmittel unter den mesomorphen oder flüssigkristallinen Farbmitteln und den mehrschichtigen Interferenzstrukturen ausgewählt ist.

12. Verfahren nach Anspruch 10 oder 11, **dadurch gekennzeichnet, dass** das goniochromatische Farbmittel unter den linearen oder cyclischen Polymeren ausgewählt ist, auf die mesomorphe Gruppen gepfropft wurden.

13. Verfahren nach einem der Ansprüche 10 bis 12, **dadurch gekennzeichnet, dass** das goniochromatische Farbmittel unter den Siliconpolymeren und Celluloseethern ausgewählt ist, auf die cholesterische Gruppen und Biphenylgruppen gepfropft sind.

14. Verfahren nach einem der Ansprüche 10 bis 13, **dadurch gekennzeichnet, dass** das goniochromatische Farbmittel eine Siliconstruktur der folgenden Formel aufweist: 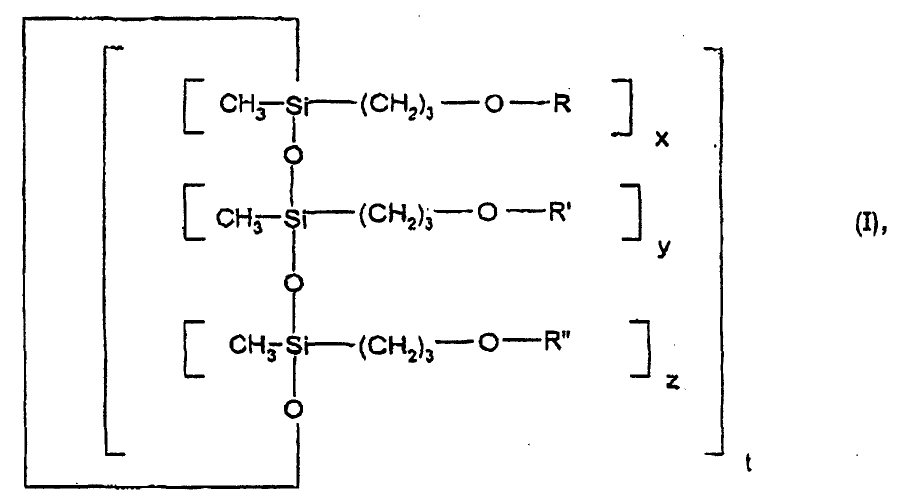 worin bedeuten:
0 ≤ x ≤ 1 (vorzugsweise 1); 0 ≤ y ≤ 1 (vorzugsweise 1); 0 ≤ z ≤ 1 (vorzugsweise 1) mit x + y + z ≠ 0; 3 ≤ t ≤ 10;
R bedeutet eine Gruppe der folgenden Formel: 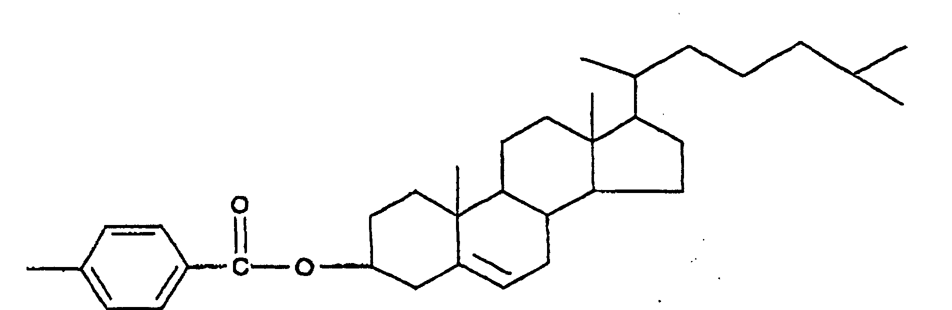
R' bedeutet eine Gruppe der folgenden Formel: 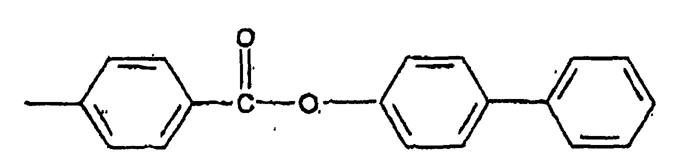
und R" bedeutet eine Gruppe der folgenden Formel:

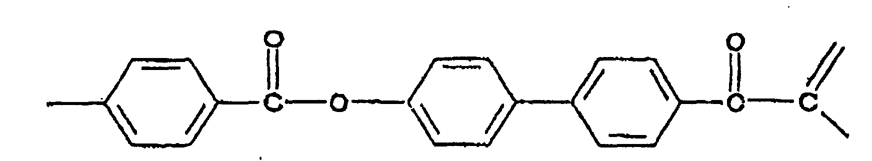

15. Verfahren nach einem der Ansprüche 10 und 11, **dadurch gekennzeichnet, dass** der goniochromatische Stoff, der eine mehrschichtige Interferenzstruktur aufweist, unter den folgenden Strukturen ausgewählt ist: Al/SiO₂/Al/SiO₂/Al; Cr/MgF₂/Al/MgF₂/Al; MoS₂/SiO₂/Al/SiO₂/MoS₂; Fe₂O₃/SiO₂/Al/SiO₂/Fe₂O₃; Fe₂O₃/SiO₂/Fe₂O₃/SiO₂/Fe₂O₃; MoS₂/SiO₂/Glimmer-Oxid/SiO₂/MoS₂; Fe₂O₃/SiO₂/Glimmer-Oxid/SiO₂/Fe₂O₃.

16. Verfahren nach einem der Ansprüche 10 bis 15, **dadurch gekennzeichnet, dass** das einfarbige Farbmittel unter den einfarbigen Farbstoffen, den einfarbigen Pigmenten und/oder Perlglanzpigmenten ausgewählt ist.

17. Verfahren nach einem der Ansprüche 10 bis 16, **dadurch gekennzeichnet, dass** der einfarbige Farbstoff unter den Oxiden von Titan, Zirconium oder Cer sowie den Oxiden von Zink, Eisen oder Chrom, Eisenblau, Ruß und den Lacken von Barium, Strontium, Calcium oder Aluminium, Sudanrot, DC Red, DC Green 6, β-Carotin, Sojaöl, Sudanbraun, DC Yellow 11, DC Violet 2, CD Orange 5, Chinolingelb, mit Titanoxid, Eisenoxid, natürlichem Pigment oder Bismutoxidchlorid überzogenem Glimmer, beispielsweise farbigen Titanglimmerpigmenten, ausgewählt ist.

18. Verfahren nach einem der Ansprüche 10 bis 17, **dadurch gekennzeichnet, dass** jedes Farbmittel 0,01 bis 98 % und besser 0,05 bis 85 % des Gesamtgewichts des Produkts ausmacht.

19. Verfahren nach einem der Ansprüche 10 bis 18, **dadurch gekennzeichnet, dass** die erste Zusammensetzung und die zweite Zusammensetzung als Make-up, Nagellack, Produkt zum Schminken des Körpers oder der Lippen, Wangenrouge oder Lidschatten konfektioniert sind.

20. Verfahren nach einem der Ansprüche 10 bis 19, **dadurch gekennzeichnet, dass** die erste Zusammensetzung und die zweite Zusammensetzung als ölige oder wässrige Lösung, öliges oder wässriges Gel, Öl-in-Wasser-Emulsion oder Wasser-in-Öl-Emulsion, Dispersion eines Öls in Wasser mit Hilfe von Vesikeln, wobei sich die Vesikel an der Grenzfläche Öl/Wasser befinden, oder als Pulver vorliegen.

21. Verfahren nach einem der Ansprüche 10 bis 20, **dadurch gekennzeichnet, dass** das kosmetisch akzeptable Medium ferner mindestens einen Bestandteil enthält, der unter den Ölen, Lösungsmitteln, Wachsen, filmbildenden Polymeren, Füllstoffen, hydrophilen oder lipophilen Wirkstoffen, Verdickungsmitteln für die wässrige Phase oder Fettphase, grenzflächenaktiven Stoffen, Antioxidantien, Parfums, Weichmachern, Neutralisationsmitteln und Stabilisierungstnitteln ausgewählt ist.

22. Verfahren nach einem der Ansprüche 10 bis 21, **dadurch gekennzeichnet, dass** die zweite Schicht diskontinuierlich ist.

23. Verfahren nach einem der Ansprüche 10 bis 22, **dadurch gekennzeichnet, dass** die zweite Schicht Motive umfasst.

24. Verfahren nach einem der Ansprüche 10 bis 23, **dadurch gekennzeichnet, dass** die zweite Schicht Motive enthält, die zufällig oder geordnet und symmetrisch oder unsymmetrisch angeordnet sind.

25. Kit zum Schminken, das eine erste Zusammensetzung mit einem kosmetisch akzeptablen Medium und mindestens einem ersten Farbmittel und eine zweite Zusammensetzung mit einem kosmetisch akzeptablen Medium und mindestens einem zweiten Farbmittel enthält, wobei ein Farbmittel ein goniochromatischer Stoff ist, der in Abhängigkeit vom Einfallswinkel des Lichts und dem Betrachtungswinkel unterschiedliche Farben erzeugen kann und unter den mehrschichtigen Interferenzstrukturen ausgewählt ist, und das andere Farbmittel ein einfarbiger Stoff ist, der eine der Farben des goniochromatischen Stoffes erzeugt.

26. Kit nach Anspruch 25, **dadurch gekennzeichnet, dass** das erste Farbmittel und das zweite Farbmittel getrennt voneinander in einer ersten bzw. einer zweiten Zusammensetzung konfektioniert sind, die ein kosmetisch akzeptables Medium enthalten.

27. Kit nach Anspruch 25 oder 26, **dadurch gekennzeichnet, dass** der goniochromatische Stoff, der eine mehrschichtige Interferenzstruktur aufweist, unter den folgenden Strukturen ausgewählt ist: Al/SiO₂/Al/SiO₂/Al; Cr/MgF₂/Al/MgF₂/Al; MoS₂/SiO₂/Al/SiO₂/MoS₂; Fe₂O₃/SiO₂/Al/SiO₂/Fe₂O₃; Fe₂O₃/SiO₂/Fe₂O₃/SiO₂/Fe₂O₃; MoS₂/SiO₂/Glimmer-Oxid/SiO₂/MoS₂; Fe₂O₃/SiO₂/Glimmer-Oxid/SiO₂/Fe₂O₃.

28. Kit nach einem der Ansprüche 25 bis 27, **dadurch gekennzeichnet, dass** das einfarbige Farbmittel unter den einfarbigen Farbstoffen, einfarbigen Pigmenten und/oder Perlglanzpigmenten ausgewählt ist.

29. Kit nach einem der Ansprüche 25 bis 28, **dadurch gekennzeichnet, dass** der einfarbige Farbstoff unter den Oxiden von Titan, Zirconium oder Cer sowie den Oxiden von Zink, Eisen oder Chrom, Eisenblau, Ruß und den Lacken von Barium, Strontium, Calcium oder Aluminium, Sudanrot, DC Red 17, DC Green 6, β-Carotin, Sojaöl, Sudanbraun, DC Yellow 11, DC Violet 2, CD Orange 5, Chinolingelb, und mit Titanoxid, Eisenoxid, natürlichem Pigment oder Bismutoxidchlorid überzogenem Glimmer, beispielsweise farbigen Titanglimmerpigmenten, ausgewählt ist.

30. Kit nach einem der Ansprüche 25 bis 29, **dadurch gekennzeichnet, dass** es Mittel umfasst, um die erste Zusammensetzung und die zweite Zusammensetzung auf die Haut und/oder die Schleimhäute und/oder die Hautanhangsgebilde aufzubringen.

31. Kit nach Anspruch 30, **dadurch gekennzeichnet, dass** diese Mittel unter den Pinseln, Füllern, Bürsten, Federn und Stiften ausgewählt sind,

32. Kit nach einem der Ansprüche 25 bis 31, **dadurch gekennzeichnet, dass** die erste Zusammensetzung und die zweite Zusammensetzung in verschiedenen Abteilungen oder Behältern konfektioniert sind.

33. Kit nach einem der Ansprüche 25 bis 32, **dadurch gekennzeichnet, dass** das kosmetisch akzeptable Medium ferner mindestens einen Bestandteil enthält, der unter den Ölen, Lösungsmitteln, Wachsen, filmbildenden Polymeren, Füllstoffen, hydrophilen oder lipophilen Wirkstoffen, Verdickungsmitteln für die wässrige Phase oder die Fettphase, grenzflächenaktiven Stoffen, Antioxidantien, Parfums, Weichmachern, Neutralisationsmitteln und Stabilisatoren ausgewählt ist.

34. Kit nach einem der Ansprüche 25 bis 33, **dadurch gekennzeichnet, dass** die Zusammensetzungen als ölige oder wässrige Lösung, öliges oder wässriges Gel, Öl-in-Wasser-Emulsion, Wasserin-Öl-Emulsion, Pulver oder Dispersion eines Öls in Wasser mit Hilfe von Vesikeln vorliegen, wobei die Vesikel sich an der Grenzfläche Öl/Wasser befinden.

35. Kit nach einem der Ansprüche 25 bis 34, **dadurch gekennzeichnet, dass** die Zusammensetzungen als Make-up, Nagellack, Produkt zum Schminken des Körpers oder der Lippen, Wangenrouge oder Lidschatten vorliegen.

36. Verwendung des Produkts nach einem der Ansprüche 1 bis 9, um in Abhängigkeit vom Betrachtungswinkel Motive auf der menschlichen Haut und/ oder den Lippen und/oder den Hautanhangsgebilden erscheinen oder verschwinden zu lassen.

37. Geschminkter Träger, der eine erste Schicht einer ersten Zusammensetzung mit mindestens einem ersten Farbmittel und eine zweite Schicht einer zweiten Zusammensetzung enthält, welche nur zum Teil auf der ersten Schicht abgeschieden wurde und mindestens ein zweites Farbmittel enthält, wobei ein Farbmittel ein goniochromatischer Stoff ist, der befähigt ist, in Abhängigkeit vom Einfallswinkel des Lichts und Betrachtungswinkel unterschiedliche Farben zu erzeugen, und das andere Farbmittel ein einfarbiger Stoff ist, der eine der Farben des goniochromatischen Stoffes erzeugt.

38. Träger nach Anspruch 37, **dadurch gekennzeichnet, dass** er in Form von falschen Nägeln, falschen Wimpern oder Perücken vorliegt.

39. Träger nach Anspruch 37 oder 38, **dadurch gekennzeichnet, dass** die zweite Schicht nicht kontinuierlich ist.

40. Träger nach einem der Ansprüche 37 bis 39, **dadurch gekennzeichnet, dass** die zweite Schicht Motive aufweist, die zufällig oder geordnet verteilt sind.

## Claims

1. Cosmetic makeup product comprising a cosmetically acceptable medium, at least one first colorant and at least one second colorant, one of the colorants being a goniochromatic colorant able to produce different colours depending on the light incidence and viewing angle, selected from multilayer interference structures, and the other colorant being a monochromatic colorant which produces one of the colours of the goniochromatic colorant.

2. Product according to Claim 1, **characterized in that** the goniochromatic colorant comprises a multilayer interference structure selected from the structures Al/SiO₂/Al/SiO₂/Al; Cr/MgF₂/Al/MgF₂/Al; MoS₂/SiO₂/Al/SiO₂/MoS₂; Fe₂O₃/SiO₂/Al/SiO₂/Fe₂O₃; Fe₂O₃/-SiO₂/Fe₂O₃/SiO₂/Fe₂O₃; MoS₂/SiO₂/oxide mica/SiO₂/MoS₂; Fe₂O₃/SiO₂/oxide mica/SiO₂/Fe₂O₃.

3. Product according to either of the preceding claims, **characterized in that** the monochromatic colorant is selected from monochromatic dyes, monochromatic pigments and/or nacreous pigments.

4. Product according to one of the preceding claims, **characterized in that** the monochromatic colorant is selected from titanium oxide, zirconium oxide and cerium oxide, and also zinc oxide, iron oxide and chromium oxide, iron blue, carbon black, and barium, strontium, calcium and aluminium lakes, Sudan Red, DC Red 17, DC Green 6, β-carotene, soya oil, Sudan Brown, DC Yellow 11, DC Violet 2, DC Orange 5, and Quinoline Yellow, mica coated with titanium oxide, with iron oxide, with natural pigment or with bismuth oxychloride, such as coloured titanium mica.

5. Product according to one of the preceding claims, **characterized in that** each colorant represents from 0.01 to 98% and, more preferably, from 0.05 to 85% of the total weight of the product.

6. Product according to one of the preceding claims, **characterized in that** it is in the form of foundation, nail varnish, a body makeup product or lip makeup product, blusher or eyeshadow.

7. Product according to one of the preceding claims, **characterized in that** the first and second colorants are packaged, respectively, in first and second compositions each comprising a cosmetically acceptable medium.

8. Product according to one of the preceding claims, **characterized in that** it is in the form of an oily or aqueous solution, an oily or aqueous gel, an oil-in-water or water-in-oil emulsion, a vesicular dispersion of oil in water, the vesicles being located at the oil/water interface, or a powder.

9. Product according to one of the preceding claims, **characterized in that** the cosmetically acceptable medium comprises, furthermore, at least one ingredient selected from oils, solvents, waxes, film-forming polymers, fillers, hydrophilic or lipophilic active substances, aqueous-phase or fatty-phase thickeners, surfactants, antioxidants, perfumes, plasticizers, neutralizing agents and stabilizers,

10. Method of making up the human skin, lips and/or epidermal derivatives, which comprises applying to the skin, lips and/or epidermal derivatives a first layer of a first composition comprising a cosmetically acceptable medium and at least one first colorant and then applying to part only of the said first layer a second layer of a second composition comprising a cosmetically acceptable medium and at least one second colorant, one of the colorants being a goniochromatic colorant able to produce different colours depending on the light incidence and the viewing angle and the other colorant being a monochromatic colorant which produces one of the colours of the goniochromatic colorant.

11. Method according to Claim 10, **characterized in that** the goniochromatic colorant is selected from mesomorphic or liquid-crystal colorants and multilayer interference structures.

12. Method according to Claim 10 or 11,
**characterized in that** the goniochromatic colorant is selected from linear and cyclic polymers onto which mesomorphic groups are grafted.

13. Method according to one of Claims 10 to 12, **characterized in that** the goniochromatic colorant is selected from silicone polymers and cellulose ethers onto which cholesteric and biphenyl-type groups are grafted.

14. Method according to one of Claims 10 to 13,. **characterized in that** the goniochromatic colorant comprises a silicone-containing structure of the following formula: 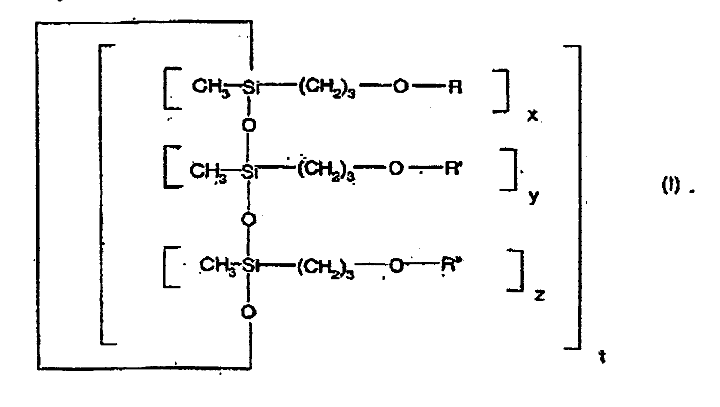 in which:
0 ≤ x ≤ 1 (preferably 1); 0 ≤ y ≤ 1 (preferably 1);
0 ≤ z ≤ 1 (preferably 1) where x + y + z ≠ 0; 3 ≤ t ≤ 10;
R denotes a group of the following formula: 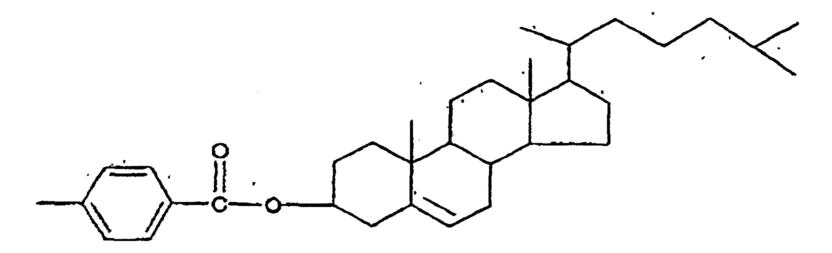
R' denotes a group of the following formula: 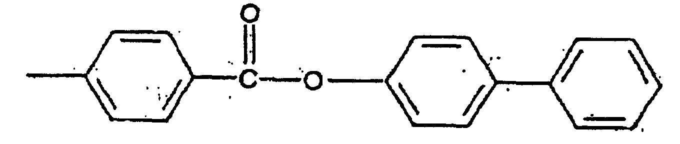 and
R" denotes a group of the following formula:

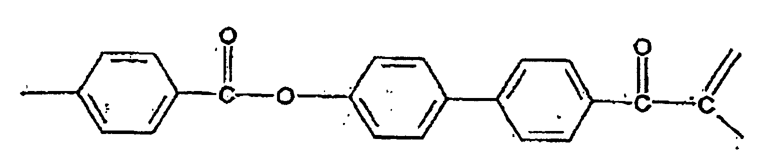

15. Method according to either of Claims 10 and 11, **characterized in that** the goniochromatic colorant comprises a multilayer interference structure selected from the structures Al/SiO₂/Al/SiO₂/Al; Cr/MgF₂/Al/MgF₂/Al; MoS₂/SiO₂/Al/SiO₂/MoS₂; Fe₂O₃/SiO₂/-Al/SiO₂/Fe₂O₃; Fe₂O₃/SiO₂/Fe₂O₃/SiO₂/Fe₂O₃; MoS₂/SiO₂/oxide mica/SiO₂/MoS₂; Fe₂O₃/SiO₂/oxide mica/SiO₂/Fe₂O₃.

16. Method according to one of Claims 10 to 15, **characterized in that** the monochromatic colorant is selected from monochromatic dyes and monochromatic pigments and/or nacreous pigments.

17. Method according to one of Claims 10 to 16, **characterized in that** the monochromatic colorant is selected from titanium oxide, zirconium oxide and cerium oxide, and also zinc oxide, iron oxide and chromium oxide, iron blue, carbon black, and barium, strontium, calcium and aluminium lakes, Sudan Red, DC Red 17, DC Green 6, β-carotene, soya oil, Sudan Brown, DC Yellow 11, DC Violet 2, DC Orange 5, and Quinoline Yellow, mica coated with titanium oxide, with iron oxide, with natural pigment or with bismuth oxychloride, such as coloured titanium mica.

18. Method according to one of Claims 10 to 17, **characterized in that** each colorant represents from 0.01 to 98% and, more preferably, from 0.05 to 85% of the total weight of the product.

19. Method according to one of Claims 10 to 18, **characterized in that** each first and second composition is in the form of foundation, nail varnish, a body makeup product or lip makeup product, blusher or eyeshadow.

20. Method according to one of Claims 10 to 19,' **characterized in that** the first and second compositions are in the form of an oily or aqueous solution, an oily or aqueous gel, an oil-in-water or water-in-oil emulsion, a vesicular dispersion of oil in water, the vesicles being located at the oil/water interface, or a powder.

21. Method according to one of Claims 10 to 20, **characterized in that** the cosmetically acceptable medium comprises, furthermore, at least one ingredient selected from oils, solvents, waxes, film-forming polymers, fillers, hydrophilic or lipophilic active substances, aqueous-phase or fatty-phase thickeners, surfactants, antioxidants, perfumes, plasticizers, neutralizing agents and stabilizers.

22. Method according to one of Claims 10 to 21, **characterized in that** the second layer is discontinuous.

23. Method according to one of Claims 10 to 22, **characterized in that** the second layer comprises motifs.

24. Method according to one of Claims 10 to 23, **characterized in that** the second layer comprises motifs in random or ordered distribution in symmetrical or asymmetrical form.

25. Makeup kit comprising a first composition comprising a cosmetically acceptable medium and at least one first colorant and a second composition comprising a cosmetically acceptable medium and at least one second colorant, one being a goniochromatic colorant which is able to produce different colours depending on the light incidence and viewing angle selected from multilayer interference structures and the other a monochromatic colorant which produces one of the colours of the goniochromatic colorant.

26. Kit according to Claim 25, **characterized in that** the first and second colorants are packaged separately in, respectively, first and second compositions comprising a cosmetically acceptable medium.

27. Kit according to Claim 25 or 26; **characterized in that** the colorant having a multilayer structure is selected from the structures Al/SiO₂/Al/SiO₂/Al; Cr/MgF₂/Al/MgF₂/Al; MoS₂/SiO₂/Al/SiO₂/MoS₂; Fe₂O₃/SiO₂/Al/SiO₂/Fe₂O₃; Fe₂O₃/SiO₂/Fe₂O₃/SiO₂/Fe₂O₃; MoS₂/SiO₂/oxide mica/SiO₂/MoS₂; Fe₂O₃/SiO₂/oxide mica/SiO₂/Fe₂O₃.

28. Kit according to one of Claims 25 to 27, **characterized in that** the monochromatic colorant is selected from dyes, pigments and/or nacreous pigments.

29. Kit according to one of Claims 25 to 28, **characterized in that** the monochromatic colorant is selected from titanium oxide, zirconium oxide and cerium oxide, also zinc oxide, iron oxide and chromium oxide, iron blue, carbon black, and barium, strontium, calcium and aluminium lakes, Sudan Red, DC Red 17, DC Green 6, β-carotene, soya oil, Sudan Brown, DC Yellow 11, DC Violet 2, DC Orange 5, and Quinoline Yellow, mica coated with titanium oxide, with iron oxide, with natural pigment or with bismuth oxychloride, such as coloured titanium mica.

30. Kit according to one of Claims 25 to 29, **characterized in that** it comprises means for applying the first and second compositions to the skin and/or mucosae and/or epidermal derivatives.

31. Kit according to Claim 30, **characterized in that** the means are selected from fine and coarse brushes, stylos, pens, quills, nibs and pencils.

32. Kit according to one of Claims 25 to 31, **characterized in that** the first and second compositions are packaged in separate compartments or containers.

33. Kit according to one of Claims 25 to 32, **characterized in that** the cosmetically acceptable medium comprises, furthermore, at least one ingredient selected from oils, solvents, waxes, film-forming polymers, fillers, hydrophilic or lipophilic active substances, aqueous-phase or fatty-phase thickeners, surfactants, antioxidants, perfumes, plasticizers, neutralizing agents and stabilizers.

34. Kit according to one of Claims 25 to 33, **characterized in that** the compositions are in the form of an oily or aqueous solution, oily or aqueous gel, oil-in-water or water-in-oil emulsion, powder, or vesicular dispersion of oil in water, these vesicles being located at the oil/water interface.

35. Kit according to one of Claims 25 to 34, **characterized in that** the compositions are in the form of foundation, nail varnish, a body makeup product or lip makeup product, blusher or eyeshadow.

36. Use of the product according to one of Claims 1 to 9, for making coloured motifs on human skin and/or lips and/or epidermal derivatives appear or disappear depending on the viewing angle.

37. Made-up substrate comprising a first layer of a first composition comprising at least one first colorant and a second layer of a second composition deposited in part only on the first layer and comprising at least one second colorant, one of the colorants being a goniochromatic colorant able to produce different colours depending on the light incidence and viewing angle and the other colorant being a monochromatic colorant which produces one of the colours of the goniochromatic colorant.

38. Substrate according to Claim 37, **characterized in that** it is in the form of false nails, false eyelashes or hairpieces.

39. Substrate according to Claim 37 or 38, **characterized in that** the second layer is discontinuous.

40. Substrate according to one of Claims 37 to 39, **characterized in that** the second layer comprises motifs in random or ordered distribution.
